Europäisches Patentamt

European Patent Office

Office européen des brevets

(19)

(11) Veröffentlichungsnummer: **0 252 169**
**A1**

(12) **EUROPÄISCHE PATENTANMELDUNG**

(21) Anmeldenummer: 86109220.3

(22) Anmeldetag: 05.07.86

(51) Int. Cl.4: **B01D 53/14** , C07C 7/11 ,
C12M 1/00

(43) Veröffentlichungstag der Anmeldung:
**13.01.88 Patentblatt 88/02**

(84) Benannte Vertragsstaaten:
**AT BE CH DE FR GB IT LI NL SE**

(71) Anmelder: **Perske, Günter**
**Im Waldblick 6**
**D-7173 Bubenorbis(DE)**

(72) Erfinder: **Perske, Günter**
**Im Waldblick 6**
**D-7173 Bubenorbis(DE)**

(74) Vertreter: **Wenzel, Joachim, Dipl.-Ing.**
**Hauptmannsreute 46**
**D-7000 Stuttgart 1(DE)**

(54) **Verfahren und Vorrichtung zur Druckregelung und unter Druck stehender Faulbehälter bei Biogasanlagen sowie Reinigung des Biogases in zwei Stufen.**

(57) Um eine einwandfreie und gefahrlose Speicherung des vom Schwefelwasserstoff befreiten Biogases zu ermöglichen, wird durch die Erfindung vorgeschlagen, daß das Biogas eine Entschwefelung unterworfen wird, indem das Biogas mittels eines (1) oder mehrerer Tauchrohre (1, 2) in Wasser (3) eingeleitet wird, welches durch ständigen Frischwasserzulauf und gleichzeitigen Ablauf ständig erneuert wird.

Dadurch erfolgt gleichzeitig eine automatische Druckregelung, ohne daß die früher verwendeten Überdruckventile benutzt werden. Die Druckregelung ist frostsicher und arbeitet störungsfrei.

FIG.1

EP 0 252 169 A1

## Verfahren und Vorrichtung zur Druckregelung und unter Druck stehender Faulbehlter bei Biogasanlagen sowie Reinigung des Biogases in zwei Stufen

Die Erfindung betrifft ein Verfahren zur Erzeugung von Biogas aus insbesondere landwirtschaftlichen Abfall-und Rohstoffen, organischen insbesondere Reststoffen (Fäkalien), wobei die Reststoffe in Gärbehältern unter der Oberfläche von dessen Füllung eingebracht werden und dort ausgegast werden, wobei die Reststoffe jeweils von unten her kontinuierlich vergleichsweise langsam eingebracht, im wesentlichen still vermischt, aber rührfrei vergärt und an der Oberfläche unter Freigabe von Biogas unter Einwirkung der Schwerkraft nach unten abgeleitet werden, wobei sie an ihrer Oberfläche mit Überdruck beaufschlagt werden und die Ausgärung schrittweise in mehreren nacheinander von den Reststoffen durchlaufenen angewärmten Stufen erfolgt.

Es ist bereits ein derartiges Verfahren bekannt, das verschiedene Vorteile gegenüber dem vorangegangenen Stand der Technik zeigt. Insbesondere kann hierdurch eine Phase geringerer Biogasproduktion bedingt durch vorangegangene Störungen des bakteriellen Umwandlungsprozesses vermieden werden. Hierbei zeigt ein erster Faulbehälter einen über ein Gegendruckventil führenden eigenen oder von einem Sammelbehälter abkuppelbaren Auslaß für die in ihm erzeugten Biogase. Durch die unterschiedliche abgestufte Einstellung der Überdruckventile bei mehreren Behältern können ferner die jeweils gewünschten Druckdifferenzen von Behälter zu Behälter eingestellt werden (Europ. Patent 0 036 065).

Es ist weiter ein Entschwefler für Biogas bekannt, der aus einem Blecheimer besteht, der unten mehrere Öffnungen aufweist und in einem Außengehäuse angeordnet ist. In dem Eimer· ist eine Reinigungsmasse enthalten. In den unteren Löchern ist der Gaszufluß vorgesehen, während oben ein Gasabfluß über der Reinigungsmasse in dem den Eimer umgebenden Gehäuse vorgesehen ist. (Beitrag des Bayerischen Staatsministeriums für Energie, Landwirtschaft und Forstwirtschaft, Dez. 1982: "Einsatzmöglichkeiten verschiedener Energieträger der Landwirtschaft" , Seite 144 und folgende, Abb. 74)

Es ist ferner eine mehrstufige Kaskaden-Gasreinigung bekannt. Es gibt auch eine thermische Entgasung, die ebenfalls mit Kaskaden arbeitet, welche mit Dampf beheizt werden. Schließlich gibt es auch die Vakuum-Entgasung.

Der Gehalt an Schwefelwasserstoff, $H_2S$, im Biogas beinhaltet ein erhöhtes Unfallrisiko. Da es - schwerer ist als Luft, sammelt es sich an tieferen Stellen und wird zur Unfallgefahr. Mögliche Gefahren ergeben sich bei Kondensatablaß vor dem Gasspeicher, durch Korrosion hervorgerufene Undichtigkeit irgendwelcher Anlageteile, besonders an Regel-und Messeinrichtungen. Ferner ist das Schwefelwasserstoff durch die Agressivität ein Störfaktor an Armaturen und Behältern und verursacht hohe Kosten bei der Überwachung und Wartung. Wenn das Biogas zur Verstromung verwendet werden soll, ist eine Entschwefelung erforderlich.

Der erwähnte bekannte Entschwefler für Biogas zeigt eine hohe Anreicherung der Reinigungsmasse mit Schwefel und kann daher zur Selbstentzündung führen. Demgemäß sind zusätzliche Schutz-und Löscheinrichtungen erforderlich. Das bekannte Verfahren der eingangs erwähnten Art zeigt auch noch den Nachteil, daß die Wärme zur Beheizung der Gärbehälter getrennt beschaft und zugeführt werden muß. Hierfür ist ein gewisser Energieaufwand erforderlich.

Ein weiterer Nachteil ergibt sich auch durch die bekannte Verwendung der Überdruckventile. Dadurch ist eine gewisse Frostgefahr und auch eine Korrosionsgefahr der mechanischen Teile vorhanden.

Der Erfindung liegt die Aufgabe zugrunde, das Verfahren und die Vorrichtung der eingangs erwähnten Art so zu verbessern, daß die genannten Nachteile vermieden und eine einwandfreie und gefahrlose Speicherung des vom Schwefelwasserstoff befreiten Biogases möglich ist. Ferner soll das Biogas auch vom Kohlendioxyd gereinigt werden, so daß das verbleibende Gas aus fast reinem Methan besteht, das einen sehr hohen Heizwert hat und eine vergleichsweise sehr viel geringere Speicherkapazität benötigt.Die bei dieser Absorbtion von Kohlendioxyd freiwerdende Wärmeenergie soll gleichzeitig zur Beheizung der Gärbehälter Verwendung finden, so daß auf diese Weise Energie eingespart wird.

Zur Lösung dieser Aufgabe ist vorgesehen, daß das Biogas nach den einzelnen Stufen einer Entschwefelung unterworfen wird, indem das Biogas mittels eines oder mehrerer Tauchrohre in Wasser eingeleitet wird, welches durch ständigen Frischwasserzulauf und gleichzeitigen Ablauf ständig erneuert wird.

Auf diese Weise erfolgt gleichzeitig eine automatische Druckregelung, ohne daß die früher verwendeten Überdruckventile benutzt werden. Dadurch ist die Druckregelung frostsicher und arbeitet störungsfrei.

Gemäß den Merkmalen des Anspruchs 2 wird erreicht, daß das gereinigte Biogas einen hohen Heizwert hat im Vergleich zum herkömmlichen Biogas und nahezu ausschließlich nur noch aus Methan besteht.

Weitere Vorteile und Einzelheiten der Erfindung ergeben sich aus der nun folgenden Beschreibung einiger Ausführungsbeispiele unter Hinweis auf die Zeichnung. In dieser zeigen:

Fig. 1 einen schematischen Schnitt durch die Anlage zur Entschwefelung des Biogases,

Fig. 2 eine schematische Darstellung der Anlage zur Absorbtion von Kohlendioxyd und

Fig. 3 eine Einzelheit der Anlage nach der Fig. 2.

Der bekannte Teil der Anlage zur Erzeugung von Biogas in den Gärbehältern ist nicht dargestellt, weil durch die Europäische Patentschrift 0 036 065 im einzelnen bekannt.

Das in den bekannten Faulbehältern, auch Fermenter oder Gärbehälter genannt, erzeugte Biogas besteht im ungünstigen Fall aus ca. 57 - 60 % Methan, $CH_4$, ca. 35 % Kohlendioxyd $CO_2$, und 0,1-0,5 % Schwefelwasserstoff, $H_2S$ sowie geringe Spuren anderer Gase, die aber unberücksichtigt bleiben können.

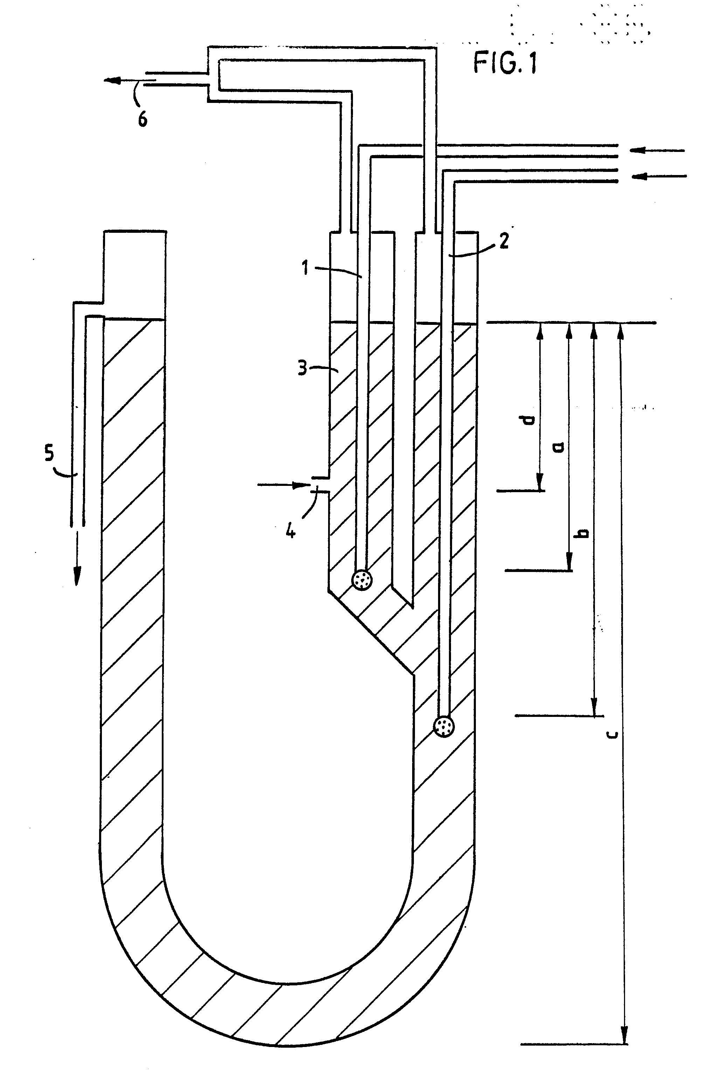

Die Fig. 1 zeigt nun eine Entschwefelungsanlage, die gleichzeitig zur Druckregelung für einen oder mehrere der erwähnten nicht dargestellten Gärbehälter bestimmt ist und einem solchen nachgeschaltet wird, wobei von Behälter zu Behälter ein abgestufter Druck des Biogases vorhanden ist.

Hierzu zeigt die Fig. 1 zunächst einen mit Wasser 3 gefüllten U-förmigen Behälter, in den rechts die beiden Tauchrohre 1 und 2 eintauchen. Natürlich kann es sich auch um eine größere Anzahl von Tauchrohren oder auch nur um ein Tauchrohr handeln. Das Tauchrohr 2 kommt von einem Behälter mit höherem Druck und führt deshalb in eine größere Tiefe b des Wassers 3. Dagegen kommt das Tauchrohr 1 von einem Behälter mit geringerem Druck und führt in eine geringere Tiefe des Wassers 3 in dem U-förmigen Behälter. Der Gasraum über dem Wasser 3 des rechten Schenkels des U-förmigen Behälters ist mit einem Gasrohr 6 zur Ableitung des von dem Schwefelwasserstoff befreiten Biogases in Richtung des Pfeiles 6 verbunden. Der linke Schenkel ist mit einem Überlaufrohr 5 verbunden, durch das das Wasser zur Entsorgung mit der schwachen Säure ständig oder in Unterbrechungen abläuft. Der rechte Schenkel zeigt einen Frischwasserzulauf 4 mit einer Eintauchtiefe d, die über der Mündung des Tauchrohres 1 liegt.

Dem Fachmann ist verständlich, daß das Biogas in dem Tauchrohr 2 einen Druck haben muß, der der Eintauchtiefe b im Wasser entspricht, so daß in dem Faulbehälter, von dem dieses Gas kommt, ständig dieser Druck mit Sicherheit aufrecht erhalten wird, ohne daß irgendein Ventil eingebaut ist.

Das Gleiche gilt natürlich für den Druck aus einem weiteren Faulbehälter in der Leitung 1, die ständig einen wesentlich geringeren Druck führt, der der Eintauchtiefe a entspricht.

Bekanntlich ist Schwefelwasserstoff, $H_2S$, im Wasser gut löslich, wo es als schwache Säure in Hydrogensulfid und Sulfidionen dissoziiert. Deshalb ist klar, daß das Biogas beim Durchgang durch das Wasser 3 vom Schwefelwasserstoffgas gereinigt wird. Gleichzeitig ist sichergestellt, daß bei einem höheren Druck in der Leitung 2 ein größerer Weg im Wasser 3 zu durchlaufen ist als bei einem geringeren Druck in der Leitung 1.

Die ständige zuverlässige Aufrechterhaltung des konstanten Druckes in dem jeweiligen Faulbehälter ist aus den bekannten Gründen erforderlich, weil nämlich gemäß dem erwähnten bekannten Verfahren die Oberfläche des nicht dargestellten Faulbehälters mit Überdruck ständig beaufschlagt werden muß.

Dem Frischlaufwasserzulauf 4 wird ständig so viel Frischwasser zugeführt, wie Schwefelwasserstoff absorbiert wird. Die gleiche Menge Wasser läuft am Überlauf Rohr 5 in Richtung des dortigen Pfeiles zur Entsorgung. Es ist ohne weiteres verständlich, daß dort genauso viel Wasser abläuft wie Frischwasser zugeführt wird, ohne daß irgendein Regelsystem oder gar ein Ventil benötigt wird.

Durch die erfindungsgemäße Vorrichtung gem. der Fig. 1 kann natürlich auch eine Druckabstufung mehrerer hintereinander geschalteter Faulbehälter vorgesehen sein, und auf diese Weise jede gewünschte Druckbeaufschlagung erreicht werden. Es kann nichts korredieren, da keine mechanischen Teile oder Armaturen die Funktion beeinträchtigen können. Diese Druckregelung kann an dem erwähnten nicht dargestellten Druck-Faulbehälter frostsicher verlegt werden und arbeitet infolge des Fehlens bewegter mechanischer Teile absolut störungsfrei.

Die erfindungsgemäße Vorrichtung ist nicht auf die Ausführungsform nach der Fig. 1 beschränkt. Zum Beispiel besteht auch die Möglichkeit, daß man auf die U-förmige Ausbildung des Behälters ganz verzichtet. Der Wasserüberlauf 5 könnte dann zum Beispiel über dem Frischwasserzulauf angeordnet sein, in-dem der Überlauf mit einer Druckbeaufschlagungsvorrichtung versehen ist, so daß der Druck höher ist als der Druck auf dem Gasometer, aber kleiner als die Druckbeaufschlagung für den ersten Fermenter. Nicht auszuschließen ist auch, daß das Wasser 3 nicht ständig, sondern von Zeit zu Zeit ausgewechselt wird.

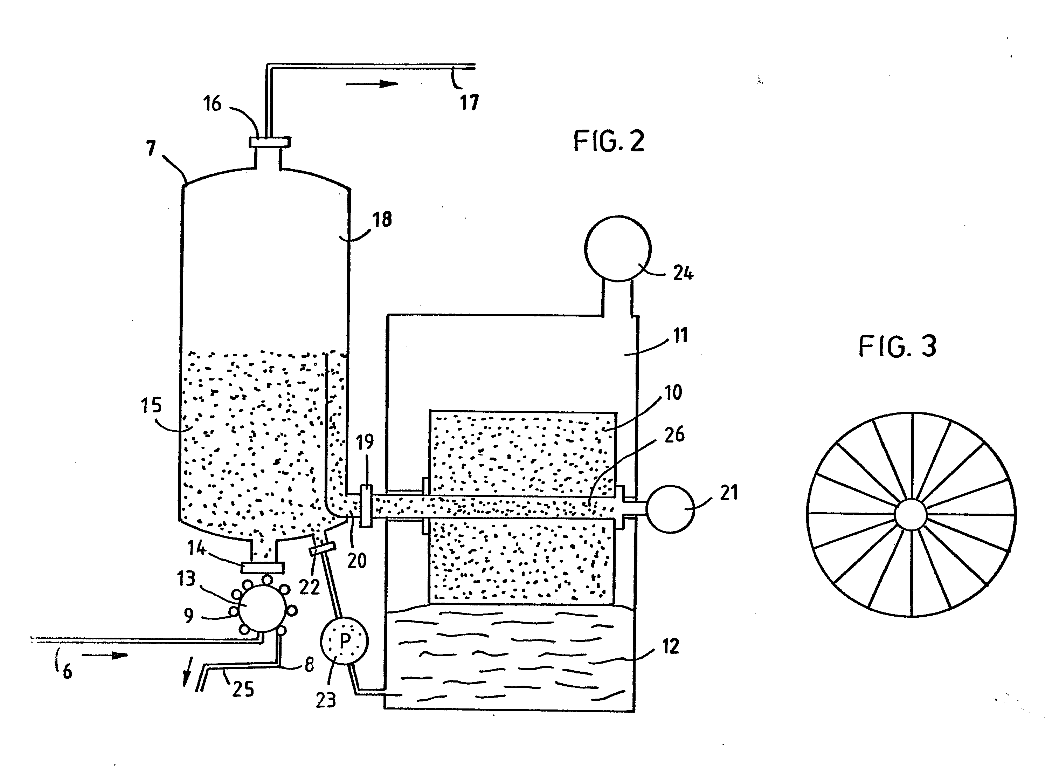

Fig. 2 zeigt die schon erwähnte Gasleitung 6, die von der Vorrichtung nach der Fig. 1 kommt. Dieses Biogas ist also bereits vom Schwefelwasserstoff befreit, und kann in einem Vorratsbehälter zwischengelagert werden. Oder es wird sofort mit dem Verdichter 13 dieses Biogas weiter über das Rückschlagventil 14 in den Druckbehälter 7 gefördert. Dabei wird es unter hohem Druck durch den unteren Bereich mit dem Wasser 15 des Behälters 7 nach oben durch das Rückschlagventil 16 in die Leitung 17 zum Endverbraucher gefördert, wenn der Druck entsprechend hoch ist oder eine Entnahme über das Ventil 16 erfolgt, das auch von Hand geöffnet werden kann.

Das unter dem hohen Druck im Behälter 7 befindliche Wasser 15 nimmt bei einem bestimmten Druck und einer bestimmten Temperatur eine bestimmte Menge Kohlendioxyd $CO_2$ auf, während sich das Methangas, $CH_4$, im oberen Behälterteil 18 sammelt. Durch das Standrohr 20 fließt das verunreinigte Wasse, wenn ein bestimmter Druck überschritten ist, über das Ventil 19 in das Entgasungselement, das in diesem Falle als drehangetriebene Lamellenwalze 10 durch den Motor 21 angetrieben wird. Das Rohr 20 hat zu diesem Zwecke im Bereich der Lamellenwalze zahlreiche Öffnungen ebenso wie die Lamellenwalze 10 selbst, die in der Fig. 3 näher dargestellt ist. Auf diese Weise gelangt das Kohlendioxydgas von innen nach außen über eine möglichst große Fläche zur Entgasung. Dabei enthält das entgaste Wasser noch den Kohlendioxydanteil beiNormaldruck. Das Wasser wird in dem Auffangbehälter 12 unterhalb der Lamellenwalze zur Wiederverwendung gesammelt und im Kreislauf durch die Leitung 8 über das Ventil 22 in den Druckbehälter 7 mittels der Pumpe 23 zurückgeführt.

Anstelle der Lamellenwalze kann auch ein Vakuumkessel herkömmlicher Bauart Verwendung finden, was dem Fachmann bekannt ist. Das dem Wasser entzogene Kohlendioxyd kann zum Beispiel kompremiert und der Industrie zugeführt werden. Hierzu kann das Wasser von Zeit zu Zeit ausgewechselt werden, es besteht aber auch die Möglichkeit, ähnlich wie gemäß derFig. 1 kontinuierlich Frischwasser zuzuführen und verunreinigtes Wasser aus dem Behälter 12 abzuführen.Dies muß nicht im enzelnen dargestellt werden, da es dem Fachmann ohne weiteres verständlich ist.

Das Ventil 19 kann auch als Druckminderventil ausgebildet sein, wodurch das unter dem hohen Druck stehende Wasser in der Leitung 20 auf Normaldruck entspannt wird.

Mittels des Sauggebläses 24 wird das Kohlendioxyd aus dem Raum 11 über der Lamellenwalze abgesaugt und einer weiteren Verwendung zugeführt.

Bekanntlich muß ein jeder Verdichter gekühlt werden, weil bei der Verdichtungsarbeit Wärme abgegeben wird. Erfindungsgemäß wird diese Wärme zur Beheizung der erwähnten nicht dargestellten Gär-oder Faulbehälter verwendet. Hierzu ist der Kühler 9 des Verdichters 13 durch eine Leitung 25 mit den erwähnten nicht dargestellten Heizvorrichtungen der Gärbehälter verbunden.

Fig. 3 zeigt einen Schnitt quer zur Längsrichtung durch die Lamellenwalze 10. Wie schon erwähnt, zeigt die Achse 26 der Lamellenwalze eine große Anzahl von Bohrungen, durch die das verunreinigte Wasser nach außen gelangen kann. Auch am Außenumfang der Lamellenwalze befinden sich entsprechende Öffnungen zum Durchlaß sowohl des Wassers als auch der Gase.

Das gereinigte Biogas besteht abgesehen von geringen Prozentanteilen anderer Gase aus fast reinem Methan, $CH_4$, und hat einen sehr hohen Heizwert von $H_u$ = 29 MJ/Nm³ -$H_o$ 35 MJ/Nm³, während im Vergleich hierzu Biogas mit 60 % Methananteil einen mittleren Heizwert von nur 21 JM/Nm³ aufweist. Im Vergleich dazu beträgt der untere Heizwert $H_u$ von 1 ltr.Öl 36 MJ und der von 1 Nm³ Stadtgas 16 MJ.

Durch die Absorbtion des Kohlendioxydes $CO_2$, verringert sich dasVolumen des Biogases um ca. 35 %, dieses hat zur Folge, daß der Gasspeicher entsprechend kleiner ausgelegt werden kann, wodurch der Aufwand erheblich verringert wird.

Wie schon erwähnt, ist die Erfindung nicht auf die dargestellten Ausführungsformen nach den Fig. 2 und 3 beschränkt, vielmehr kann anstelle der Lamellenwalze 10 auch ein nicht dargestellter herkömmlicher Vakuumkessel Verwendung finden. Die dargestellte Ausführungsform ist aber aus den genannten Gründen bevorzugt.

Somit ist der Fachmann in der Lage, im Rahmen der Ansprüche Abwandlungen der dargestellten Verfahren und Vorrichtungen auszuführen.

Ansprüche

1. Verfahren zur Erzeugung von Biogas aus insbesondere landwirtschaftlichen Abfall-und Rohstoffen, organischen insbesondere Reststoffen (Fäkalien), wobei die Reststoffe in Gärbehältern unter der Boerfläche von dessen Füllung eingebracht werden und dort ausgegast werden, wobei die Reststoffe jeweils von unten her kontinuierlich vergleichsweise langsam eingebracht, im wesentlichen still vermischt, aber rührfrei vergärt und an der Oberfläche unter Freigabe von Biogas unter Einwirkung der Schwerkraft nach unten abgeleitet werden, wobei sie an ihrer Oberfläche mit Überdruck beaufschlagt werden und die Ausgärung schrittweise in mehreren nacheinander von den Reststoffen durchlaufenen angewärmten Stufen erfolgt, dadurch gekennzeichnet, daß das Biogas nach den einzelnen Stufen einer Entschwefelung unterworfen wird, indem das Biogas mittels eines oder mehrerer Tauchrohre (1, 2) in Wasser (3) eingeleitet wird, welches durch ständigen Frischwasserzulauf (4) und gleichzeitigen Ablauf (5) ständig erneuert wird.

2. Verfahren nach Anspruch 1, dadurch gekennzeichnet, daß das Biogas (6) nach der Entschwefelung gemäß Anspruch 1 einem Verfahren zur Absorbtion von Kohlendioxyd unterworfen wird, in dem es zunächst in einem Überdruckbehälter (7) zusammen mit Wasser (8) unter Druck gesetzt und die dadurch entstehende Wärme zur Beheizung (9) der einzelnen Stufen verwendet wird, woraufhin das Wasser in eine angetriebene Lamellenwalze (10) gegeben und das Kohlendioxyd aus der Umgebung der Lamellenwalze (11) abgesaugt wird.

3. Vorrichtung zur Durchführung des Verfahrens nach Anspruch 1, dadurch gekennzeichnet, daß das oder die Tauchrohre (1, 2) in einem U-förmigen Wasserbehälter (3) mit einem Frischwasserzulauf (4) und einem Überlauf (5) angeordnet ist bzw. sind.

4. Vorrichtung zur Durchführung des Verfahrens nach Anspruch 1, dadurch gekennzeichnet, daß die Eintauchtiefe (a, b) der Tauchrohre (1, 2) unterschiedlich ist, so daß das Biogas bei entsprechend unterschiedlichen Drücken entweicht.

5. Vorrichtung zur Durchführung des Verfahrens nach Anspruch 2, dadurch gekennzeichnet, daß das von der Lamellenwalze (10) kommende Wasser in einem Behälter (12) unterhalb der Lamellenwalze aufgefangen und dem Druckbehälter (7) wieder zugeführt wird.

FIG.1

7063a

FIG. 2

FIG. 3

0 252 169

Europäisches
Patentamt

**EUROPÄISCHER RECHERCHENBERICHT**

Nummer der Anmeldung

EP 86 10 9220

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| Y | EP-A-0 036 065 (E. GÜNTER)<br>* Zusammenfassung; Abbildung 1 *<br>& DE-A-3 010 183 | 1 | B 01 D 53/14<br>C 07 C 7/11<br>C 12 M 1/00 |
| | --- | | |
| Y | DE-A-3 109 120 (H. STEFFEN)<br>* Ansprüche 1-4; Abbildung 1 * | 1 | |
| | --- | | |
| Y | DE-A-3 117 638 (H. KNEPPER)<br>* Seite 15, Absätze 6-22; Seite 17, Absätze 4-12; Abbildung * | 1 | |
| A | | 4 | |
| | --- | | |
| A | EP-A-0 180 670 (CRYOTEC ENERGY SYSTEMS)<br>* Seite 2, Absatz 16 - Seite 7, Absatz 12; Abbildung 1 * | 1 | RECHERCHIERTE SACHGEBIETE (Int. Cl.4)<br><br>B 01 D<br>C 07 C<br>C 12 M |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 12-03-1987 | PYFFEROEN K. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82